# EUROPEAN PATENT SPECIFICATION

(11) **EP 2 545 873 B1**
(45) Date of publication and mention of the grant of the patent: **29.07.2015**
(21) Application number: 11753073.3
(22) Date of filing: 11.01.2011
(51) Int. Cl.: A61B 18/12, A61B 18/18

(54) **MEDICAL DEVICE**
MEDIZINISCHE VORRICHTUNG
DISPOSITIF MÉDICAL

(30) Priority: 11.03.2010 JP 2010054847
(43) Date of publication of application: 16.01.2013
(73) Proprietor: Terumo Kabushiki Kaisha, Tokyo 151-0072 (JP)
(72) Inventor: KATOU, Yukitoshi, Ashigarakami-gun Kanagawa 259-0151 (JP)
(74) Representative: Dossmann, Gérard
(86) International application number: PCT/JP2011/050264
(87) International publication number: WO 2011/111410

(56) References cited:
- EP-A1- 0 876 804
- WO-A1-2009/028542
- JP-A- 2 304 880
- JP-A- 2 304 880
- JP-A- 2007 519 489
- JP-A- 2009 232 878
- JP-A- 2009 233 021
- JP-U- 60 021 185
- JP-Y2- 62 039 333
- US-A- 6 122 549
- US-A1- 2002 165 536
- US-A1- 2005 015 048
- US-A1- 2007 265 610
- US-A1- 2009 069 810
- US-A1- 2009 118 729
- US-A1- 2010 049 099

## Description

### TECHNICAL FIELD

The present invention relates to a medical device for heat-treating a living body and particularly relates to improvement of a hand-side steering unit for steering a medical device, and in particular to a device according to the preamble of claim 1, such as it is known from WO-A1-2009/028542.

### BACKGROUND ART

Recently, as a medical treatment device with respect to a patent foramen ovale (hereinafter, referred to as PFO: Patent Foramen ovale) which is a cardiogenic factor of a stroke and a migraine headache, there has been proposed a device described in Patent Document 1.

This PFO closing device is a device in which an apparatus is inserted into the foramen ovale from the right atrium toward the left atrium, a foramen ovale valve is pulled so as to close the foramen ovale, the foramen ovale valve and the atrial septum secundum are sandwiched by a pair of electrodes, and the biological tissue is to be fused by applying electric energy from both the electrodes.

In this device, there are used clamping means in which one side thereof is made of a sticking member composed of a needle electrode and the other side thereof is made of a sandwiching member for sandwiching the foramen ovale valve and the atrial septum secundum with respect to the sticking member and the sticking member is stuck into the foramen ovale valve and thereafter, the foramen ovale valve and the atrial septum secundum are sandwiched with respect to the sandwich member which is the other electrode, electric energy is applied to the biological tissue, and the fusion is carried out.

This device can be used also in case of closing defects such as a congenital atrial septum secundum defect (ASD), a PFO, a ventricular septal defect (VSD) and a patent ductus arteriosus (PDA), and it is a device having high general versatility and in particular, foreign substances are not indwelled in the body, the constitution becomes simple, also the procedure becomes easy and the foramen ovale valve and the atrial septum secundum can be fused reliably.

Meanwhile, when applying electric energy to the clamping means (electrode) which is exposed inside the blood, it sometimes happens that thrombi will easily be attached to the clamping means and it is not desirable for the electric energy to be carelessly applied to the electrode for fusing the biological tissue.

The present invention was invented in order to solve the problem mentioned above and has an object to provide a medical device having high safety in which it is possible to depress the electric energy from being applied carelessly.

### Prior-art Document

### Patent Document

Patent Document 1: WO2007/100067

### DISCLOSURE OF THE INVENTION

A medical device of the present invention, which achieves the aforementioned object, is a medical device including a heating unit provided on a distal side of a catheter to heat a biological tissue and a hand-side steering unit provided at a proximal portion of the catheter to steer advance and retraction operation with respect to the catheter at the heating unit, characterized in that the hand-side steering unit comprises: a main body portion interlocked with the catheter; a slide portion which is interlocked with the heating unit and which is approachable and separable with respect to the main body portion; a guide unit which is interlocked movably with one of the main body portion and the slide portion and also which is fixed on the other thereof; and an input connector which is provided at the main body portion, the slide portion or the guide unit, which is electrically connected with the heating unit and also with which an output connector for supplying electric energy is connectable, wherein one of the members, within the main body portion, the slide portion and the guide unit, which is different from the member provided with the input connector is arranged at a position for hindering connection between the input connector and the output connector and also, there is provided a connection adjusting unit which is a notch or a through-hole for enabling connection between the input connector and the output connector when the heating unit moved to a position at which the biological tissue is heatable by moving the slide portion with respect to the main body portion.

In a medical device relating to the present invention, one of the members, within the main body portion, the slide portion and the guide unit, which is different from the member provided with the input connector is arranged at a position for hindering connection between the input connector and the output connector and also, there is provided a connection adjusting unit so as to enable connection between the input connector and the output connector when the heating unit moves to a position at which the biological tissue is heatable after the guide unit is moved with respect to the main body portion or the slide portion, so that it is possible to reliably prevent a careless electric energy application until the heating unit moves as far as a position at which the biological tissue is heatable and it is possible to improve safety.

If it is constituted such that the connection adjusting unit has a size allowing connection between the input connector and the output connector when the slide portion lies in a relative position within a certain range with respect to the main body portion, it is possible to improve safety by suppressing careless electric energy application while responding flexibly to the biological tissue which has individual difference.

If it is constituted such that at the main body portion, at the slide portion or at the guide unit, there is provided a switch for carrying out electrical connection or release between the heating unit and the input connector by the movement of the slide portion; and corresponding to the position of the slide portion at which electrical connection is carried out by the switch, the connection adjusting unit is provided such that connection between the input connector and the output connector becomes possible, it is possible to suppress careless electric energy application also by means of a switch and it is possible to improve safety much more.

If it is constituted such that for the connection adjusting unit and the output connector, there is provided fixing means for fixing the movement of the slide portion with respect to the main body portion by being contacted with each other when the output connector is connected to the input connector, it is possible to suppress the movement of the heating unit after the output connector is connected to the input connector and there can be realized more desirable heating.

If it is constituted such that for the guide unit, there is provided an indicating portion in which there is a notification so as to coincide with a predetermined position of the main body portion or of the slide portion in a state in which the connection between the input connector and the output connector is possible, the operator can easily understand that electric energy application to the heating unit is possible and further, it is possible to improve the safety.

### BRIEF DESCRIPTION OF DRAWINGS

FIG. 1 is a schematic cross-sectional view showing a PFO closing device relating to this exemplified embodiment;
FIG. 2 is a main portion perspective view showing the PFO closing device relating to this exemplified embodiment;
FIG. 3 is a cross-sectional view of a catheter distal portion taken along a 3-3 line in FIG. 2;
FIG. 4 is a plan view showing a hand-side steering unit of the PFO closing device relating to this exemplified embodiment;
FIG. 5 is a cross-sectional view of the hand-side steering unit taken along a 5-5 line in FIG. 2;
FIG. 6 is an enlarged cross-sectional view of the hand-side steering unit taken along the 5-5 line in FIG. 2;
FIG. 7 is an enlarged cross-sectional view of the hand-side steering unit showing an occasion when a slide portion is moved backward;
FIG. 8 is a cross-sectional view showing an interlock mechanism taken along an 8-8 line in FIG. 4;
FIG. 9 is a cross-sectional view of a lock & unlock mechanism portion taken along a 9-9 line in FIG. 4;
FIG. 10 is a cross-sectional view taken along a 10-10 line in FIG. 9;
FIGS. 11 are plan views showing the hand-side steering unit on an occasion when a needle steering lever is steered, in which FIG. 11A shows a state before the steering and FIG. 11B shows a state after the steering;
FIGS. 12 are enlarged plan views showing the hand-side steering unit on an occasion when a slide portion is moved backward, in which FIG. 12A shows a state in the midst of the backward moving and FIG. 12B shows a state after the backward moving;
FIG. 13 is an enlarged plan view showing the hand-side steering unit on an occasion when an output connector is connected to an input connector of the hand-side steering unit;
FIG. 14 is a plan view of the hand-side steering unit on an occasion when a needle steering lever is moved backward;
FIG. 15 is a cross-sectional schematic diagram in which a main steering rod is inserted into a foramen ovale;
FIG. 16 is a cross-sectional schematic diagram in a state in which a foramen ovale valve is held and a sticking unit is stuck;
FIG. 17 is a cross-sectional schematic diagram in which a foramen ovale valve and an atrial septum secundum are sandwiched by a sticking unit and a sandwich member;
FIGS. 18A to 18D are schematic diagrams showing operation states of the PFO closing device respectively;
FIG. 19 is a plan view showing a guide bar and the output connector according to a modified example of this exemplified embodiment;
FIG. 20 is a perspective view showing another exemplified embodiment to which the present invention is applied; and
FIG. 21 is a perspective view showing still another exemplified embodiment to which the present invention is applied.

### MODE FOR CARRYING OUT THE INVENTION

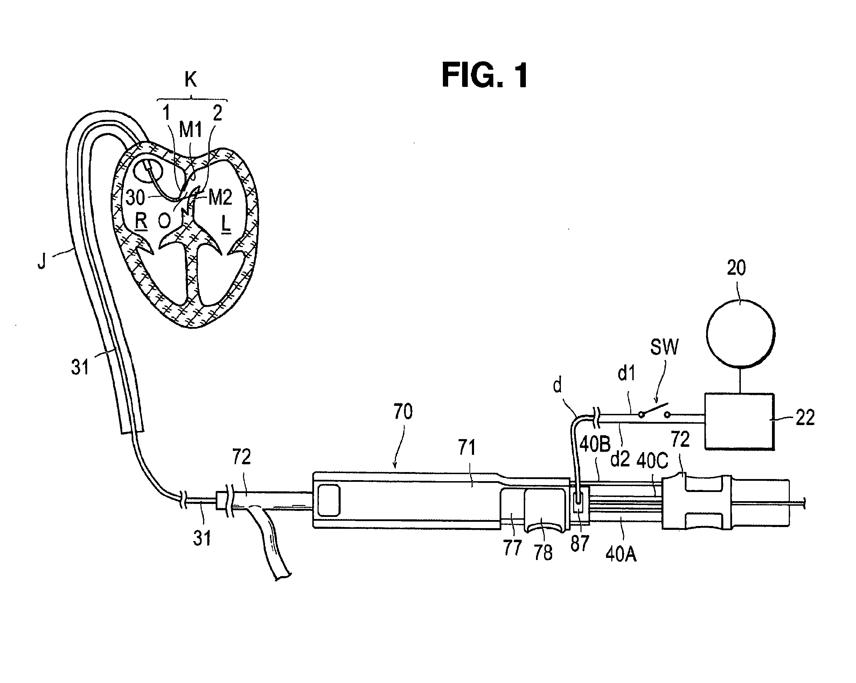

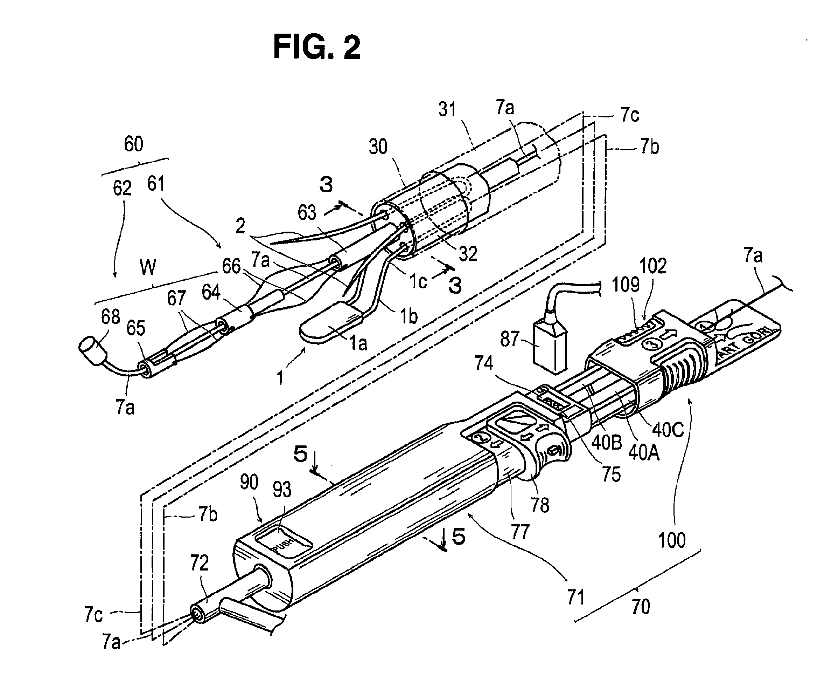

A medical device of this exemplified embodiment is a PFO closing device, and an outline thereof will be explained first with reference to FIGS. 1 to 3. Note that in FIG. 2, due to limitations of space, there is a description in a state in which only a hand-side steering unit 70 is demagnified.

The PFO closing device includes a hand-side steering unit 70 provided on the proximal side, a guiding catheter 31 whose proximal end is interlockable with the hand-side steering unit 70, a catheter 30 whose proximal end is attached to the hand-side steering unit 70 and which is provided in the guiding catheter 31 and, clamping means K which is provided at a distal portion of the catheter 30 and which sandwiches a foramen ovale valve M2 and an atrial septum secundum M1, energy supply means 20 for supplying electric energy which fuses or necrotizes a biological tissue M (generic term of M1, M2) of a portion sandwiched by the clamping means K, and positioning hold means 60 (see FIG. 2) for performing the surgical procedure by the clamping means K stably and also accurately. Note, in the following explanation, that the side of the hand-side steering unit 70 of the device is referred to as "proximal side" and the side of the clamping means K is referred to as "distal side".

On an occasion of using the device, first, the guiding catheter 31 is inserted, for example, from a femoral vein J, and this guiding catheter 31 is inserted in a state in which, in the inside thereof, the clamping means K provided at the distal end of the catheter 30 is stored together with the catheter 30. After the distal end reaches the region of the heart at which the procedure is to be executed, the clamping means K is protruded from the catheter 30 by steering the hand-side steering unit 70 and the tissues of the atrial septum secundum M1 and the foramen ovale valve M2 of the heart are sandwiched in which there occurs a defect O of a foramen ovale (hereinafter, it sometimes happens that this is referred to simply as foramen ovale O). In this sandwiched state, the clamping means K is supplied with electric energy, both the tissues are heated and fused, and the defect O is closed. More specifically, the clamping means K functions as a heating unit. Note, in the drawing, that "L" denotes a left atrium and "R" denotes a right atrium.

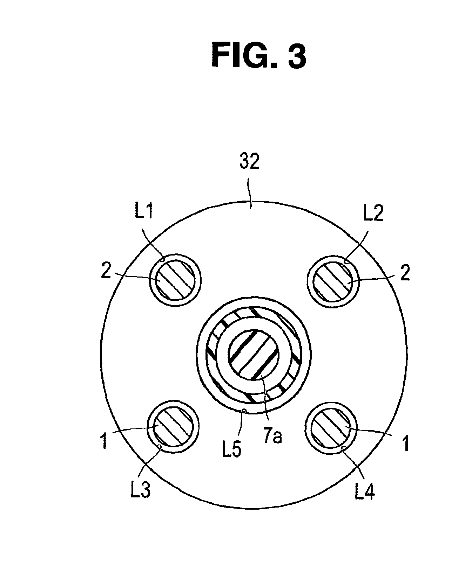

The clamping means K is constituted by a sandwich member 1 directly contacting with one side surface of the atrial septum secundum M1 and a sticking member 2 which is stuck into the foramen ovale valve M2. The sandwich member 1 is constituted, as shown in FIG. 2, by a flat-plate portion 1a having a flat plate shape as a whole and a pair of wire portions 1b connected to the proximal portion; and the flat surface position thereof is restricted by lumens L3, L4 (see FIG. 3) of a distal end tip 32 fixed at the distal end of the catheter 30. Also, the sandwich member 1 is connected with one line of steering cord 7b on the proximal side of the wire member portion 1b formed in a U-shape, and by advancing and retracting the steering cord 7b in the axial direction, the sandwich member 1 protrudes from the distal end tip 32 and forms a predetermined sandwich width with respect to the sticking member 2, is displaced so as to sandwich the biological tissue M by approaching toward the sticking member 2 side when entering into the distal end tip 32, and so on.

On the other hand, the sticking member 2 is held by lumens L1, L2 (see FIG. 3) formed in the distal end tip 32 so as to be movable forward and backward in a state in which the flat surface position thereof is restricted and also, the distal portion thereof is constituted so as to be retractable from the distal end tip 32 by operating a steering cord 7c connected to the proximal side formed in a U-shape.

The sticking member 2 is given elasticity such that very fine two needle-shaped members whose cross-sections perpendicular to the axes are circles and whose distal ends are sharply pointed are mutually separated, and also, the distal end thereof is widely opened when protruded. Also, it is allowed for the number of needle members either to be one piece or to be three pieces or more.

The sandwich member 1 and the sticking member 2 both function as electrode members (heating units), and the steering cord 7b, 7c which push-out the sandwich member 1 and the sticking member 2 retractively from the catheter 30 (see FIG. 2) are connected electrically with the energy supply means 20 through an input connector 75 which passes through inside the catheter 30 and which is provided at the hand-side steering unit 70 mentioned later, an output connector 87 which is a plug fitted with that (see FIG. 1), and a conduction wire d (common designation of d1, d2) connected with the electrode terminal of the output connector 87 and a control unit 22. Also, either one of the conductive wires d1 and d2 (conductive wire d1 in this exemplified embodiment) is provided with a foot switch SW to be installed at the operator's feet in order to ON/OFF control the electric current from the energy supply means 20. Note that it is allowed, instead of the foot switch SW, to employ a switch which can easily be operated on the hand-side.

The hand-side steering unit 70 is a unit for steering the clamping means K composed of a pair of electrode members which sandwich the biological tissue M lying in the vicinity of a defect existing in the biological tissue such that the clamping means K is pushed out freely retractively from the distal end of the catheter 30, and, here, there are provided means described below and the like collectively such that it is possible to carry out all operations within that small area without moving a hand much.

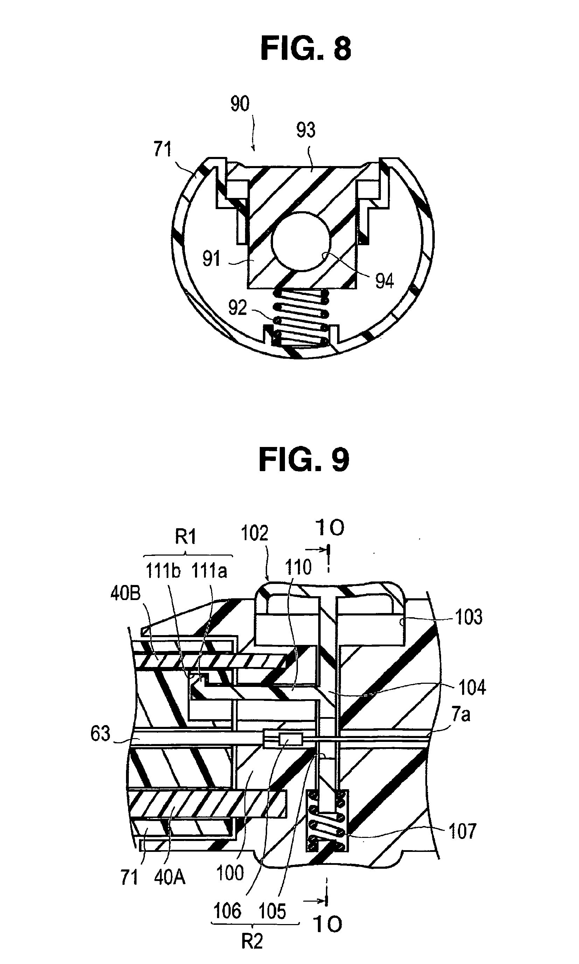

In other words, the hand-side steering unit 70 is provided, as shown in FIG. 2, with a needle steering lever 78 for steering the sticking member 2 which is one electrode member, a slide portion 100 for steering the sandwich member 1 which is the other electrode member, a main steering rod 7a which is a rod for assisting the steering of the clamping means K and which is passed through movably in the axial direction inside the hand-side steering unit 70 and the catheter 30, a pusher piece 109 which operates a lock & unlock mechanism 102 for locking/unlocking the slide movement of the slide portion 100 (see FIG. 9) and concurrently, which locks the movement of the main steering rod 7a in the axial direction, and an input connector 75 provided with an electrode terminal to be connected with energy supply means 20 for applying thermal energy.

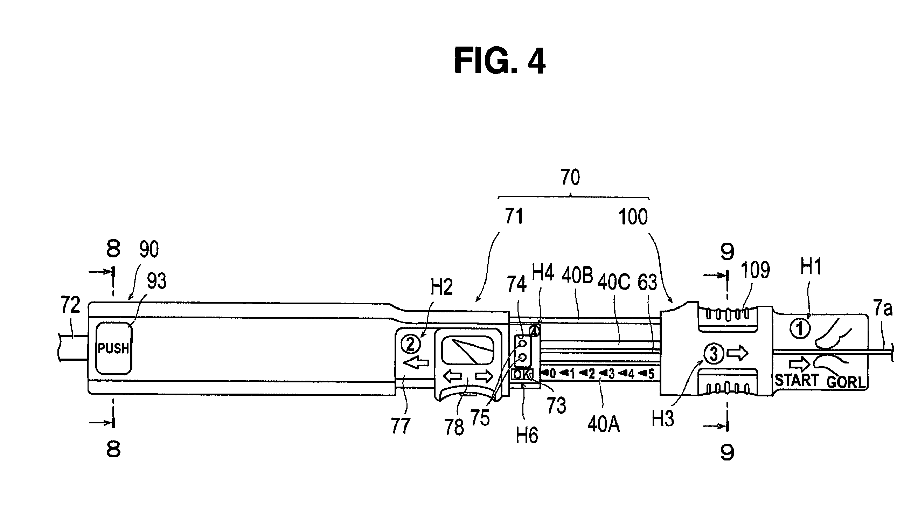

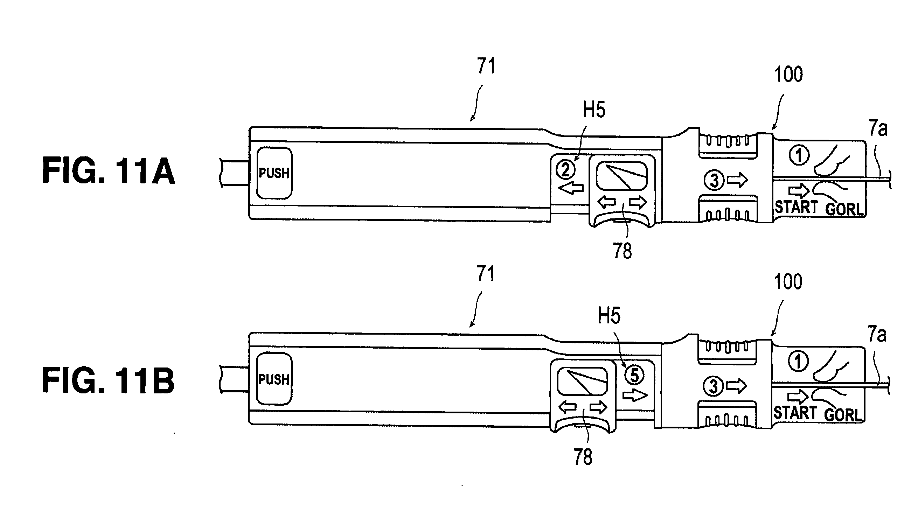

As shown in FIG. 4, the hand-side steering unit 70 is provided, in order to make processes in various kinds of procedures visible, with a process indicating portions H (common designation of H1 to H5) which are given various indications at the surface portion thereof for guiding the operator so as to carry out a correct operation (see FIG. 11B for the process indicating portion H5).

The process indicating portion H is constituted by an indicating portion H1 for a rod traction process of operating the pusher piece 109 and exerting traction on the main steering rod 7a; an indicating portion H2 for a sticking process in which the sticking member 2 sticks the biological tissue; an indicating portion H3 for a slide portion movement process which moves the slide portion 100 slidingly and carries out sandwiching or release of the biological tissue; an indicating portion H4 for a connection process of connecting the input connector 75 with the energy supply means 20; and an indicating portion H5 (see FIG. 11B) for a sticking unit moving-back process of moving the sticking member 2 backward from the biological tissue, and there are provided indications for imaging respective processes by using graphic indications, numbers and arrows of movement directions respectively. In the process indicating portion H, the color by which each steering-member is colored and the color of the arrow indicating the movement direction of each steering-member are made to be the same in order to make it easy to understand the steering of the steering-member.

In this manner, when a process indicating portion H is provided for the hand-side steering unit 70 such that the orders and movement directions of various kinds of processes become visible, it is not necessary for the operator to be perfectly familiarized with the sequence of the processes beforehand, the sequence can be understood easily if seeing the process indicating portion H, it is possible to reduce a mental burden at the time of use and to carry out the procedure smoothly and also reliably, and further, safety is also improved. Note that it is allowed for the process indicating portion H not only to make an indication by using graphic indication but also to make an indication in an itemization manner on the surface of the hand-side steering unit 70.

Note that with respect to the operation immediately before carrying out the connection process, it is needless to say that not only there is employed a case of sandwiching the biological tissue by the clamping means K but also there may be employed other procedure. In other medical devices, there are employed various kinds of operations for the operation immediately before carrying out connection between the energy supply means 20 and the input connector 75, and also to those cases, this exemplified embodiment is applicable.

With relation to the needle steering lever 78, when moving the sticking member 2 in the sticking direction (from the state shown in FIG. 11A to the state shown in FIG. 11B), it is constituted such that there appear an indication of the subsequent moving direction and a number indicating the order of the operation process from the lower surface of the needle steering lever 78. In this manner, a fool proof function, in other words a safety function, which is designed so as not to incur a dangerous situation even if the operator carries out an erroneous operation, is exerted and the reliability and safety of the procedure are heightened.

Further, when explaining the hand-side steering unit 70 in detail, the hand-side steering unit 70 includes, as shown in FIG. 2, a main body portion 71 on the side to which the guiding catheter 31 is interlocked and a slide portion 100 which is interlocked with the proximal side of the main body portion 71 through guide bars (guide units) 40A, 40B, 40C so as to approach and separate with respect to the main body portion 71, and on the upper surface of the main body portion 71, there is provided a needle steering lever 78 which steers the sticking member 2.

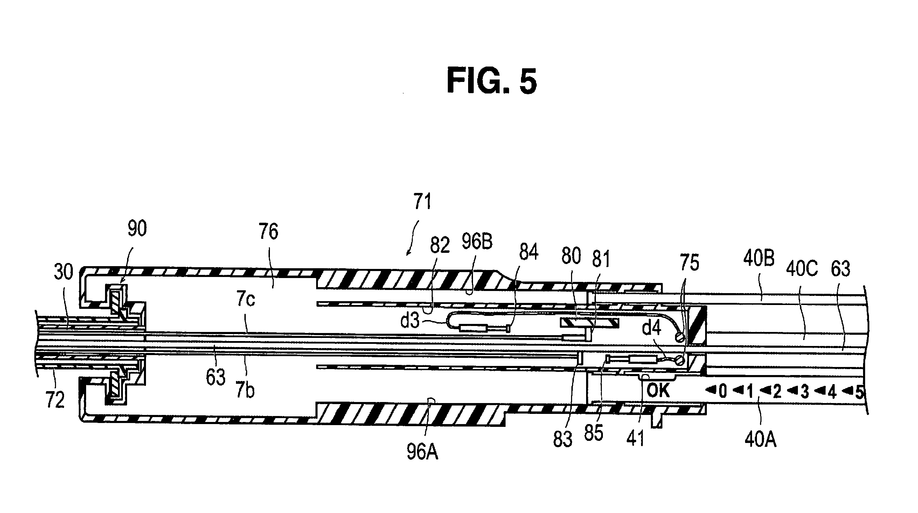

On the surface side (upper surface side) of the main body portion 71, there is formed a concave portion 77 as shown in FIG. 4 and the needle steering lever 78 is provided here slidably in the lengthwise direction (see outline arrow) . As shown in FIG. 5, the needle steering lever 78 includes a bracket 80 through which a slit (not shown) formed at the main body portion 71 is inserted and which is protruded so as to reach an inner space 76, and with respect to this bracket 80, there is interlocked an L-shaped terminal 81 which is provided on the proximal side of the steering cord 7c for the sticking member 2. Therefore, when sliding the needle steering lever 78 (see FIG. 4) along the slit, it is constituted such that the terminal 81 slides, as shown in FIG. 5, along a guide groove 82 which is formed inside the main body portion 71 and the sticking member 2 is advanced and retracted through the steering cord 7c.

Approximate at the center of the inner space 76 of the main body portion 71, the main tube 63 explained in detail later is passed through. The proximal side of the main tube 63 is interlocked with the slide portion 100 by an adhesive agent or the like (see FIG. 9), and in accordance with the slide operation of the slide portion 100, it slides by being guided by the main body portion 71.

To the main tube 63 inside the inner space 76, a terminal 83 is attached in the vicinity of the right end and it is constituted such that also the terminal 83 slides along with the sliding of the main tube 63. To the terminal 83, a steering cord 7b is connected and the steering cord 7b is inserted passing through a side portion of the main tube 63. At the movement termination end positions of these terminals 81, 83, there are provided contact members 84, 85 functioning as switches. It is needless to say that the electric system of the sticking member 2 and the electric system of the sandwich member 1 are insulated so as not to be conducted.

The contact members 84, 85 are connected to both electrodes of the input connector 75 by means of conductive wires d3, d4 and are constituted so as to contact with terminals 81, 83 respectively before reaching the movement-completion positions of the terminal 81, 83 which move along with the movement of the steering cord 7c for the sticking member 2 and the steering cord 7b for the sandwich member 1.

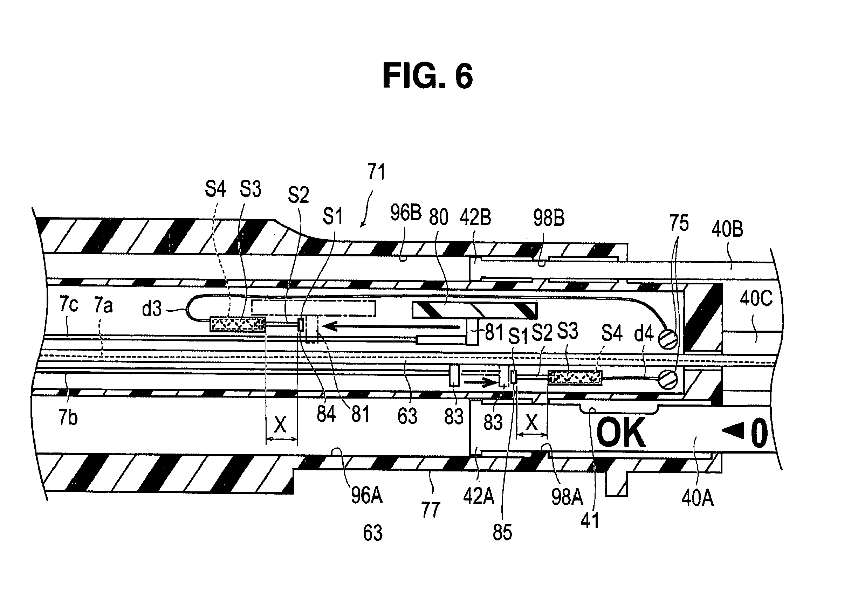

The contact members 84, 85 will be explained in further detail. As shown in FIG. 6, the contact members 84, 85 include attaching portions S1 contacting with the terminals 81, 83; leg portions S2 protruding from the attaching portions S1; tube shaped collars S3 inside which protrusion ends of the leg portions S2 are housed; and springs S4 for springing the leg portions S2 toward the outside thereof. Therefore, each attaching portion S1 is protruded by the spring S4 on a steady basis, but it is pushed by the terminal 81, 83 and moves backward, so that, as shown in the drawing, there is a conductable range X of a predetermined length.

By employing such a configuration, even if the sticking state of the sticking member 2 or the sandwiching state of the sandwich member 1 becomes different because of a difference in the thickness and/or the shape of the foramen ovale valve M2 or the like depending on an individual person and the movement-completion positions of the terminals 81, 83 become all different, the contact members 84, 85 contact with the terminals 81, 83 reliably, and electrical conduction becomes possible and reliability of the procedure is secured. In addition, there also exists a mechanism having a constitution in which an electric contact state is to be formed slidably, but in comparison with such a mechanism, the contact between the contact members 84, 85 and the terminals 81, 83 becomes reliable, furthermore, breakdown becomes less likely, and also with respect to the slide operation of the terminals 81, 83, the frictional resistance force becomes less and lighter.

However, it is not necessary for the pairs of the contact members 84, 85 and the terminals 81, 83 to be all in an elastic contact state and it is allowed if at least only one side thereof is in that state and it is allowed for the other side thereof to be constituted in a usual contact state. Alternatively, it is allowed for both the sides thereof to be constituted in a usual contact state without providing the pairs of the contact members 84, 85 and the terminals 81, 83.

Guide bars 40A, 40B are provided in the insides of grooves 96A, 96B inside the main body portion 71, and a guide bar 40C is provided in the inside of a groove which is not shown. As shown in FIG. 6, protrusion portions 42A, 42B for stopping dropout are provided at ends on one side of the guide bars 40A, 40B and they are constituted so as to stop by abutting to stopper portions 98A, 98B provided in the grooves 96A, 96B respectively.

The main steering rod 7a is a rod which is provided inside the main tube 63 and which has a function of assisting the operation of the clamping means K by being traction-operated in the axial direction, and it is constituted so as to be rotatable by 360 degrees centering around the axial line inside the main tube 63. If the main steering rod 7a is rotatable by 360 degrees, it is possible for the rod to be inserted through the foramen ovale O by inserting the distal end of the main steering rod 7a as far as the vicinity of the foramen ovale O and by positionally displacing this rod in a rotational manner. According to this result, even if the state of the foramen ovale O is deformed variously, it is possible for the distal end of the device to be inserted through the foramen ovale O regardless of the shape state thereof and it is possible to achieve easiness and speediness of the procedure.

At the distal portion of the main body portion 71, there is provided a push button 93 of an interlock mechanism 90 (see FIG. 2). The interlock mechanism 90 is a mechanism for making detachment & attachment of a Y connector 72 easy with respect to the main body portion 71 and in a state of pushing down the push button 93, when the pressing force to the push button 93 is released after fitting a flange portion provided at the proximal portion of the Y connector 72 into an insertion hole formed in the main body portion 71, as shown in FIG. 8, the flange portion of the Y connector 72 engages with an engagement hole 94 of a slide member 91. Then, the slide member 91 is sprung by a spring member 92, there is exerted a drop-out stopping function of the flange portion, and there is employed a constitution in which by pushing down the push button 93 further, it becomes possible for the Y connector 72 to be detachable.

Note that at the distal end of the hand-side steering unit 70, as shown in FIG. 2, it is preferable to interlock the Y connector 72 into which it is possible to inject a contrast agent or the like by means of the interlock mechanism 90, but in a case in which the Y connector 72 is not used, it happens that a guiding catheter 31 having a flange portion is to be directly interlocked with the main body portion 71. Note that it is allowed for the Y connector 72 to be provided at an arbitrary position of the guiding catheter 31.

At the proximal portion of the main body portion 71, there is provided a connection hole 74 corresponding to the exterior shape of the output connector 87 and inside this connection hole 74, there is arranged an electrode terminal of the input connector 75.

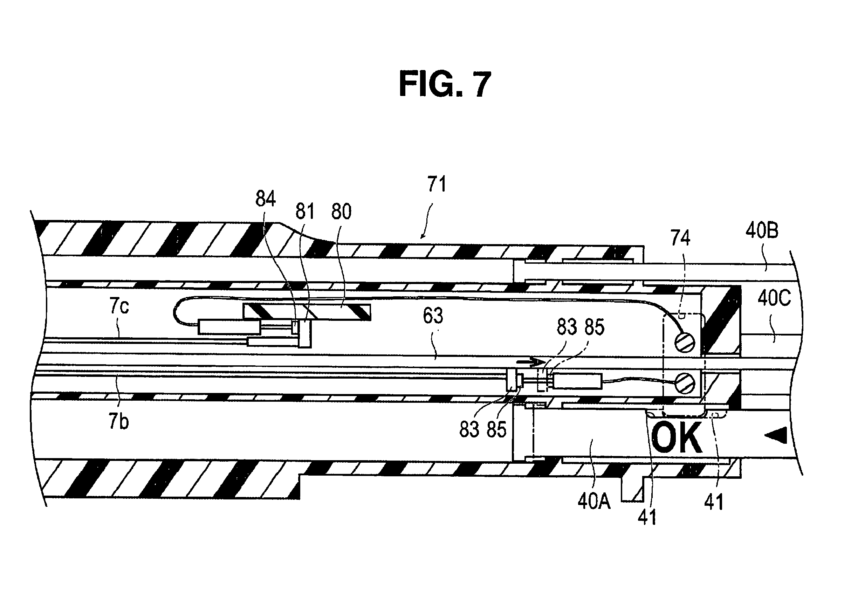

The guide bar 40A is arranged such that a portion of the lateral side thereof enters into the connection hole 74, and the guide bar 40A which has entered into the connection hole 74 hinders insertion of the output connector 87 into the connection hole 74 and prevents connection of the output connector 87 with the input connector 75. At a portion of the lateral side of the guide bar 40A, there is formed a cutout portion 41 (connection adjusting unit) and as shown in FIG. 7, the output connector 87 becomes connectable with the input connector 75 by a mechanism in which this cutout portion 41 coincides with the connection hole 74.

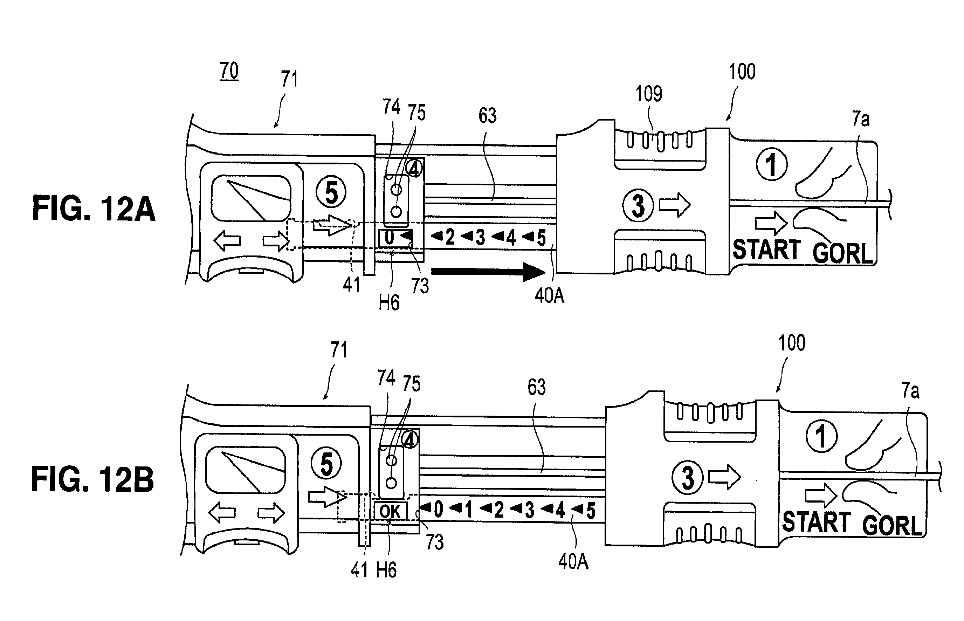

The guide bar 40A and the main tube 63 are both fixed to the slide portion 100, so that when, as shown in FIGS. 12A and 12B, the slide portion 100 is moved backward in the proximal direction, as shown by the dashed-dotted lines in FIG. 6, the main tube 63 slides inside the main body portion 71 together with the guide bar 40A, and the terminal 83 fixed on the main tube 63 contacts with a contact member 85, and the sandwich member 1 and the input connector 75 are connected electrically. This contact member 85 has a conductable range X of a predetermined length as mentioned above, so that the cutout portion 41 is formed on the guide bar 40A by such a size that makes it possible for the output connector 87 to be connected to the input connector 75 within this conductable range X.

More specifically, in FIG. 7, the terminal 83, the contact member 85 and the cutout portion 41 which are shown by dashed-dotted lines indicate a transition from a state in which by moving the slide portion 100 backward, the terminal 83 and the contact member 85 are first contacted (see terminal 83, contact member 85 and cutout portion 41 which are shown by solid lines in FIG. 7) to a state in which the slide portion 100 is further moved backward as much as the conductable range X, and it is constituted, within this range, such that the cutout portion 41 coincides with the connection hole 74 and the guide bar 40A does not protrude into the connection hole 74 and the output connector 87 (see FIG. 1) is connectable to the input connector 75 without being hindered by the guide bar 40A.

Owing to the fact that such a constitution is provided, there is realized a constitution in which the connection between the energy supply means 20 and the input connector 75, which is the most essential procedure and for which carefulness is required, can be carried out only after the sandwiching of the biological tissue M is completed, and safety of procedure is heightened by itself regardless of the physical or mental state of the operator.

Also, as shown in FIG. 4, the main body portion 71 is provided with a window 73 which is established adjacent to the input connector 75. Then, on the guide bar 40A, there is notified an "OK" indicating portion H6 (see FIG. 6) in the vicinity the cutout portion 41 and further, from the "OK" indicating portion H6, numbers (1 to 5) are sequentially notified at a constant pitch together with triangle arrows.

When pulling-in and withdrawing the positioning hold means 60 into the inside of the catheter 30 by moving the slide portion 100 backward from the main body portion 71, it is constituted for the hand-side steering unit 70 such that the numbers notified on the guide bar 40A appear at the window 73 sequentially so as to be counted down and finally, the "OK" indicating portion H6 appears at the window 73. The terminal 83 contacts with the contact member 85 within the conductable range X, so that the whole "OK" indicating portion H6 appears at the window 73 within this conductable range X and it is constituted, in case of deviating from the conductable range X, such that the "OK" indicating portion H6 does not appear at the window 73.

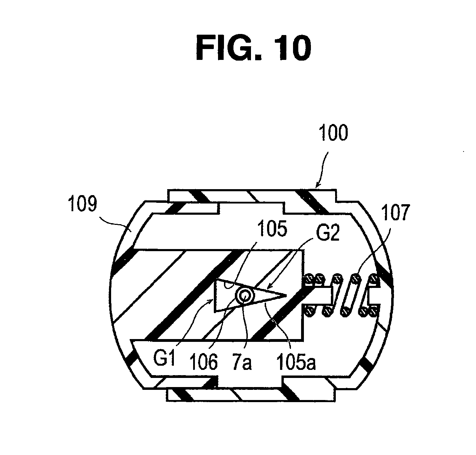

The lock & unlock mechanism 102 shown in FIGS. 9 and 10 is provided at the slide portion 100 and by pressing the pusher piece 109, the mechanism locks & unlocks the slide movement of the slide portion 100 and concurrently, locks & unlocks the axial direction movement of the main steering rod 7a.

The lock & unlock mechanism 102 is provided together with a first lock portion R1 for the slide portion, which interlocks the slide portion 100 and the main body portion 71 by sliding an operation member 104, makes the slide movement possible by releasing the lock and so on; and a second lock portion R2 for the main steering rod, which temporarily stops the advance and retraction steering in the axial line direction of the main steering rod 7a when positioning hold means 60 mentioned later which is provided at the distal portion of the main steering rod 7a holds or positions the biological tissue M.

The first lock portion R1 is constituted by an operation member 104 provided freely slidably inside a slide hole 103 which is formed at the slide portion 100 and a restricting rod 110 which is provided integrally with the operation member 104 and which restricts the movement of the slide portion 100 with respect to the main body portion 71. A reference numeral "107" in FIGS. 9 and 10 indicates a spring.

The restricting rod 110 is provided with an engagement protrusion 111a, which is engaged with an engagement concave portion 111b of the main body portion 71, at the distal end thereof, so that when the operation member 104 is pressed, the engagement between the engagement protrusion 111a and the engagement concave portion 111b is released and it becomes possible for the slide portion 100 to slide with respect to the main body portion 71. Therefore, if the slide portion 100 is operated backward, it is possible to operate the sandwich member 1 adjacently with respect to the sticking member 2 through the steering cord 7b. In addition, the operation member 104 is provided also with the second lock portion R2 and caused by the pressing of the operation member 104, also the second lock portion R2 is to be released.

If the release of the first lock portion R1 and the release of the second lock portion R2 are linked by operating the pusher piece 109 and the operation member 104 in this manner, it is possible to link the storing operation of the sandwich member 1 into the inside of the catheter and the operation for surely setting the main steering rod 7a to be in a straight shape when pulling out the long main steering rod 7a from the left atrium side, it is possible to prevent a traction operation in a state in which the main steering rod 7a having a possibility of damaging the biological tissue M is curved and/or a moving-back operation of the sandwich member 1 which is in a sandwiching state, and it is possible to prevent a situation in which the biological tissue M is damaged or fractured.

On the other hand, the second lock portion R2 for the main steering rod 7a is constituted by a locking portion 105 formed at the operation member 104 and a large diameter portion 106 formed at the main steering rod 7a. With respect to the second lock portion R2, in order to stop the advance and retraction steering of the main steering rod 7a in the axial line direction temporarily, the locking portion 105 provided at the operation member 104 is formed as a wedge-shaped through-hole having a wide width portion G1 and a narrow width portion G2. If the wedge-shaped through-hole is employed in this manner, by only moving the main steering rod 7a inside the through-hole, the sandwiching of the large diameter portion 106 becomes stronger and even if pressurizing means or the like is not provided separately, it is possible to hold the main steering rod 7a to the fixing position and it is possible to carry out the procedure easily, safely and also reliably.

In case of carrying out a procedure, a stick operation by the sticking member 2 is carried out after the positioning hold means 60 carries out holding and/or positioning of the biological tissue M, and the holding and/or the positioning of the biological tissue M is carried out by exerting traction on the main steering rod 7a. Even if the holding and/or the positioning of the biological tissue M is carried out by exerting traction on the main steering rod 7a, it is not possible to carry out the stick operation as long as the holding state and/or the positioning state are/is not maintained. Therefore, even if the second lock portion R2 engages the large diameter portion 106 with the locking portion 105 (depending on the situation, entrance edge portion 105a of a through-hole) when traction-steering the main steering rod 7a, the main steering rod 7a is temporarily brought into a locked state and the hand grasping the main steering rod 7a is released, it makes it possible to maintain the holding state and/or the positioning state and to carry out only the sticking operation by the sticking member 2 independently.

Also, for the second lock portion R2, it is possible to heighten usability of the steering depending on the position at which the large diameter portion 106 is provided. For example, in case of carrying out traction of the main steering rod 7a in the direction of being pulled out from the slide portion 100, if the large diameter portion 106 is pressed into the locking portion 105 and is locked at the position where the pulling-out is stopped, it is possible to lock the main steering rod 7a in a hold state of the hold portion 62 and to maintain the hold state. Also, if the lock is released, the distal portion of the main steering rod 7a becomes straight in shape automatically by the elasticity of the elastic wires 66, 67 in the hold portion 62 and the hold state of the foramen ovale valve M2 can be released easily.

The energy supply means 20 shown in FIG. 1 is means for supplying electric energy to the clamping means K and this has a publicly-known system constitution, so that detailed explanation thereof will be avoided, but from a viewpoint of easiness of control, it is preferable to employ electrical means regardless of direct current power source or alternate current power source. However, it is not limited only by this and it is allowed to employ any kind of means if it is possible to supply energy by which the foramen ovale valve M2 and the atrial septum secundum M1 sandwiched by the clamping means K can be fused by using heat and can be pressed and bonded by adhesive factors such as collagen, elastin and the like. For example, it is also possible to use ultrasound, laser, microwave or high frequency wave and the like.

The positioning hold means 60 is, as shown in FIG. 2, constituted by, in general, a positioning portion 61 for positioning the sticking member 2 with respect to the foramen ovale O and a hold portion 62 for holding the foramen ovale valve M2 in a non-retractable manner with respect to the sticking direction of the sticking member 2 and normally, it is housed inside the guiding catheter 31, but at the time of use, it is pushed out from the guiding catheter 31 by steering the main steering rod 7a and the main tube 63 as shown in the drawing.

If explained in further detail, in the center lumen L5 formed at the distal end tip 32, there are provided the main tube 63 and the main operation rod 7a which is provided freely advancingly and retractingly in the axis direction inside the main tube 63. The main tube 63 is a tube whose proximal side is held fixedly at the slide portion 100 and which exerts a center axis like function of this device, and also, it is a tube for reinforcing the catheter 30 and further, it is also a tube for pulling and withdrawing the positioning hold means 60 into the catheter 30. The main steering rod 7a passes through inside the main tube 63 from the distal end of the catheter 30, passes through an internal path of the slide portion 100 and is protruded from the rear end thereof.

At the distal portion of the main tube 63, there is provided the positioning portion 61 of the positioning hold means 60. The positioning portion 61 is a portion for positioning the sticking member 2 with respect to the foramen ovale O and is, as shown in FIG. 2, constituted by a pair of first elastic wires 66 which are operated expandably and contractably by the steering of the main operation rod 7a. The proximal end of the first elastic wire 66 is mounted on the outer surface of the main tube 63 and the distal end thereof is mounted on the proximal side of the intermediate sleeve body 64 inside which the main steering rod 7a is inserted.

The positioning portion 61 protrudes the main operation rod 7a from the distal end of the main tube 63, displaces the first elastic wires 66 outward by making the proximal end attached to the main tube 63 serve as a supporting point depending on the operation for advancing and retracting the main operation rod 7a in the axis direction, depresses the inner edge of the foramen ovale O with approximately equal elastic force by the respective first elastic wires 66, and aligns the sticking member 2 with respect to the foramen ovale O. In other words, there is exerted a function in which the sticking member 2 positioned between both the first elastic wires 66 is positioned at a central portion of the foramen ovale O.

On the other hand, the hold portion 62 is a portion which holds the sticking member 2 from the rear surface side so as to stick the foramen ovale valve M2 easily and includes, as shown in FIG. 2, a bump member 68 provided at the distal portion of the main steering rod 7a, a distal end sleeve body 65 and a pair of second elastic wires 67 by which the intermediate sleeve body 64 and the distal end sleeve body 65 are interlocked. The bump member 68 is fixed on the distal end of the main steering rod 7a and with respect to the distal end sleeve body 65 and the intermediate sleeve body 64, the main steering rod 7a is inserted into the inside thereof, and with respect to the second elastic wire 67, the proximal end thereof is welded to the distal end of the intermediate sleeve body 64 and the distal side thereof is welded to the distal end sleeve body 65.

The second elastic wire 67 and the bump member 68 for interlocking the intermediate sleeve body 64, the distal end sleeve body 65 and both the sleeve bodies 64, 65 constitute a curving mechanism W which curves or bends the distal portion of the main steering rod 7a.

The curving mechanism W is a mechanism used for holding the foramen ovale valve M2. When the sticking member 2 sticks the foramen ovale valve M2, the sticking becomes easy if the thin foramen ovale valve M2 is held from the rear surface side thereof. Therefore, the curving mechanism W is constituted such that the second elastic wire 67 is curved or bent between the bump member 68 and the distal side of the first elastic wire 66 by moving the main steering rod 7a backward in the axial direction and the foramen ovale valve M2 is held from the rear surface side by the bump member 68 and the distal end sleeve body 65. In other words, the curving mechanism W is constituted such that the distal portion of the main steering rod 7a is curved or bent by making the distal side of the first elastic wire 66 mounted on the main tube 63 serve as a supporting point.

However, it is necessary for the curving mechanism W of the hold portion 62 to be constituted so as to be curved and hold the foramen ovale valve M2 after the first elastic wire 66 of the positioning portion 61 aligns and positions the sticking member 2 with respect to the foramen ovale O, so that it is necessary for the first elastic wire 66 to deform in advance of the second elastic wire 67, and therefore, in this exemplified embodiment, both the elastic members are made different in rigidity.

When the slide portion 100 is advanced and retracted with respect to the main body portion 71, it is possible for the main tube 63 fixed firmly to the slide portion 100 to be pulled into the inside of the lumen L5 in the center of the catheter 30 and along with this operation, it is possible to withdraw the whole positioning hold means 60 into the inside of the catheter 30.

Next, an operation of this exemplified embodiment will be explained.

### (Preprocess)

The operator inserts an introducer (dilator & long sheath) from the femoral vein. The distal end of the long sheath is made to reach the left atrium L by way of the right atrium R and thereafter, the dilator is pulled out from the long sheath.

The pusher piece 109 of the first lock portion R1 in the lock & unlock mechanism 102 is pressed to the inward of the slide portion 100, and the operation member 104 is lowered inside the slide hole 103 and the restriction of the restricting rod 110 is removed. Thus, the slide portion 100 gets into a movable state with respect to the main body portion 71. Note that a portion of the lateral side of the guide bar 40A enters into the connection hole 74, thereby hindering connection of the output connector 87 to the input connector 75, and unexpected power supply from the energy supply means 20 is suppressed reliably and safety is secured.

When the slide portion 100 is moved backward with respect to the main body portion 71 and concurrently, the needle steering lever 78 is also moved backward, there is obtained a state in which the wire member portion 1b of the sandwich member 1, the sticking member 2 and the like are stored inside the catheter 30.

In this state, the catheter is inserted into the inside of the long sheath and is made to reach as far as the left atrium L passing through the femoral vein J & the right atrium R.

When the distal end of the catheter 30 reaches the left atrium L, the slide portion 100 is made to progress with respect to the main body portion 71. Thus, the flat-plate portion 1a of the sandwich member 1 protrudes from the distal end of the catheter 30 through the terminal and the steering cord 7b, and also, the main tube 63 is moved forward and concurrently, the pusher piece 109 of the lock & unlock mechanism 102 is pressed and a state is brought about in which the large diameter portion 106 of the main steering rod 7a does not abut the narrow width portion G2 of the through-hole 105 formed in the operation member 104, in other words, in which the second lock portion R2 is brought into an unlocked state and the main steering rod 7a is brought into a free state.

Then, from the distal end of the main tube 63, the distal end of the main steering rod 7a is protruded from the distal end sleeve body 65 and inserted into the pulmonary vein Q. it is possible for this protrusion state to be confirmed visibly from the outside because an X-ray impermeable marker is provided on the bump member 68. The main steering rod 7a is rotatable by 360 degrees, so that it can progress while rotating the main steering rod 7a and it can be inserted easily through the pulmonary vein Q.

In a state in which the main steering rod 7a is inserted in the pulmonary vein Q, the hand-side steering unit 70 is pulled until the sandwich member 1 reaches the right atrium R. At that time, the distal end of the main steering rod 7a protrudes from the distal end sleeve body 65 and is inserted inside the left atrium L.

(1) The traction processes of the main steering rod (Note that in the drawing, the sequence of the processes is indicated by numbers with circles, but in the description, they are indicated by numbers in parentheses. Hereinafter, similar indication is employed.)

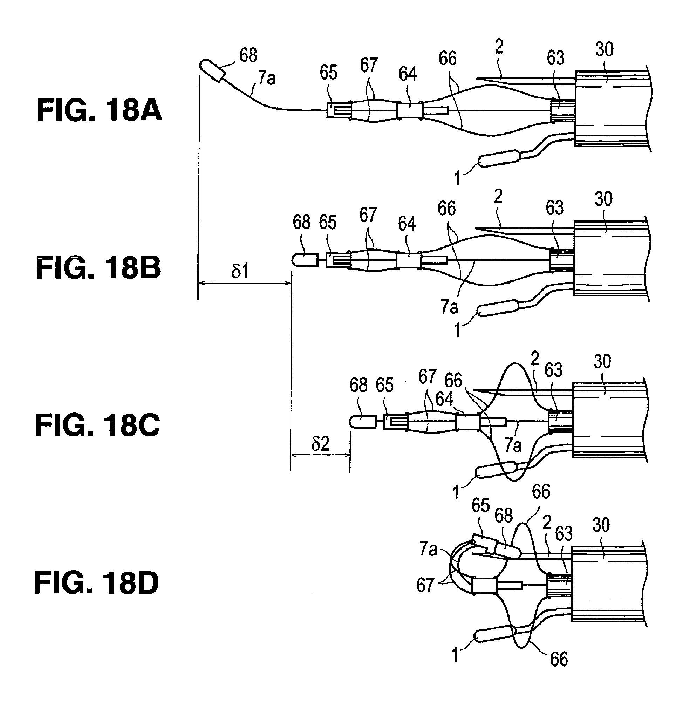

As shown in FIG. 4, at the indicating portion H1 of the rod traction process, there is applied an indication of exerting traction on the main steering rod 7a by fingers together with an indication of the number (1). In accordance with this indication, after confirming the distal end position of the main steering rod 7a, the operator makes, as shown in FIG. 18B, the main steering rod 7a move backward until the bump member 68 at the distal end of the main steering rod 7a abuts the distal end sleeve body 65 (the move-back amount is "δ1" in FIG. 18B).

When the main steering rod 7a is moved backward, the large diameter portion 106 is also moved backward and in the lock & unlock mechanism 102, the operation member 104 is biased upward by the spring force of the spring 107 unless pressing the pusher piece 109, so that the main steering rod 7a is regularly compression-held between the narrow width portion G2 of the wedge-shaped through-hole 105 and the inner circumferential surface of the internal path Qb and therefore, it is possible, with respect to the moving-back of the main steering rod 7a, to carry out the pulling operation smoothly. Then, the main body portion 71 is steered and the second elastic wire 67, the sandwich member 1 and the sticking member 2 are positioned in the vicinity of the foramen ovale valve M2, and the whole hold portion 62 is inserted to the left atrium L side.

When the main steering rod 7a is moved backward further (the moving-back amount is "52" in FIG. 18C), this steering force of moving backward is transmitted to the first elastic wire 66, whose proximal end is mounted on the main tube 63, through the bump member 68, the distal end sleeve body 65, the second elastic wire 67 and the intermediate sleeve body 64 by the main steering rod 7a, and the first elastic wire 66 is deformed protrusively in an arc shape toward the outward in the diameter direction as shown in FIG. 18C. However, at this time point, the second elastic wire 67 is not deformed.

According to this result, it happens that the first elastic wire 66 deforms while pressing and expanding the rim portion of the foramen ovale O, so that the sticking member 2 provided just near the first elastic wire 66 is aligned with respect to the foramen ovale O and the sticking member 2 is positioned at the center of the foramen ovale O.

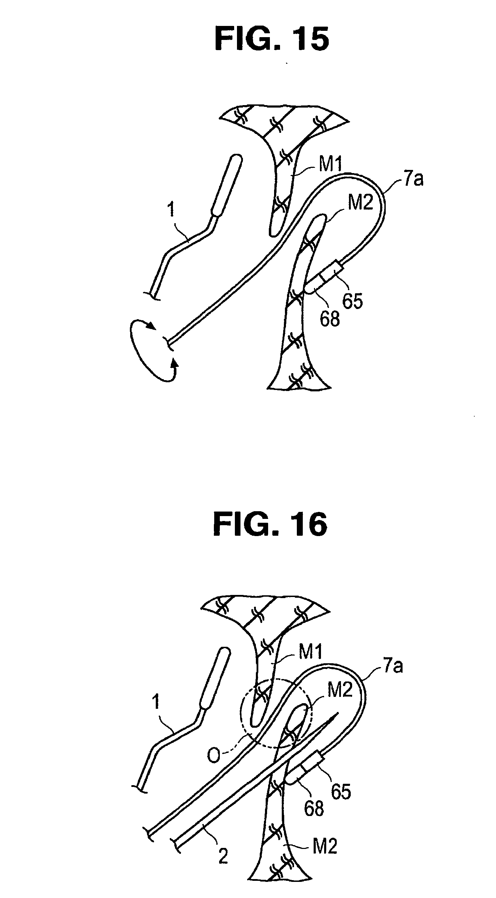

When the main steering rod 7a is steered further so as to move backward and when the rear end of the intermediate sleeve body 64 abuts the distal end of the main tube 63 as shown in FIG. 18D, the first elastic wire 66 does not deform much and the second elastic wire 67 on the distal side deforms protrusively in an arc shape toward the outward in the diameter direction by the steering force. According to this result, as shown in FIG. 15, the bump member 68 and the distal end sleeve body 65 curves in the inside of the left atrium L so as to approach to the sticking member 2, so that it happens that the bump member 68 and the distal end sleeve body 65 abut the surface on the left atrium side of the foramen ovale valve M2 and hold this.

In the second lock portion R2 for the lock & unlock mechanism 102 shown in FIGS. 9 and 10, the large diameter portion 106 is pressed into the locking portion 105 which is a wedge-shaped through-hole and the main steering rod 7a is locked. According to this result, even if the operator releases a hand from the main steering rod 7a, the hold state is maintained reliably and it never happens that the hold of the foramen ovale valve M2 is loosened and it is possible for the operator to make the needle steering lever 78 progress only by a single hand.

### (2) Sticking Process

When the needle steering lever 78 is made to progress in the arrow direction (see FIG. 11), the sticking member 2 protrudes from the distal end of the catheter 30 through the steering cord 7c and as shown in FIG. 16, the sticking member 2 is stuck into a predetermined position of the foramen ovale valve M2. There is no fear that there occurs a situation such as impossibility of sticking caused by looseness of the holding of the foramen ovale valve M.

When the sticking member 2 is moved toward the sticking direction, it is constituted for the hand-side steering unit 70, as shown in FIG. 11B, such that an indication of a next moving direction and a number indicating the sequence of the operation process appear from the bottom surface thereof. By doing so, it is possible to prevent forgetting to pull out the sticking member 2 after the sticking and the reliability and safety of the procedure is heightened.

The position of the sticking member 2 is set by the positioning hold portion 62, so that there is no fear of deviation and also, when sticking the sticking member 2 once, the position of the sticking member 2 becomes an almost fixed position in relation to the foramen ovale valve M2. Therefore, the sticking operation becomes extremely easy for the operator.

When the sticking is completed, the slide portion 100 is made to progress further with respect to the main body portion 71. Thus, the flat-plate portion 1a of the sandwich member 1 protrudes from the distal end of the catheter 30 through the terminal and the steering cord 7b.

Then, at the hand-side steering unit 70, as shown in FIG. 7, the terminal 81 mounted on the needle steering lever 78 progresses and contacts the member 84 and there can be obtained an electrically conductive state between the sticking member 2 and the input connector 75.

### (3) Movement Process of Slide Portion

When the flat-plate portion 1a comes to a position facing the atrial septum secundum M1, the slide portion 100 is moved backward from the main body portion 71 as shown in FIG. 12A. Even at this time point, a portion of the guide bar 40A has entered into the connection hole 74, thereby hindering connection of the output connector 87 to the input connector 75, so that safety is secured.

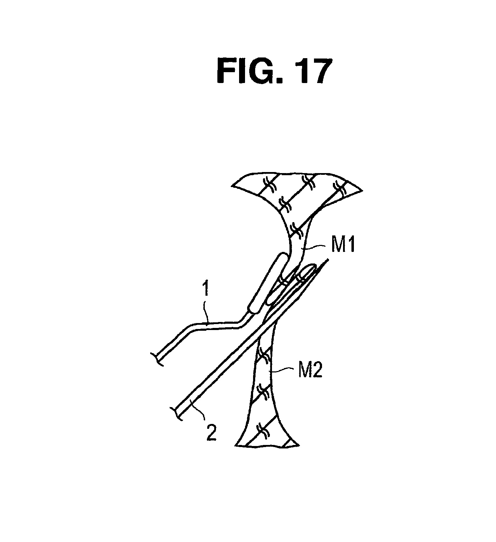

Caused by the moving-back of the slide portion 100, the flat-plate portion 1a is moved backward through the steering cord 7b shown in FIG. 2, receives an influence exerted when a bend portion 1c of the wire member portion 1b enters into the inside of the lumen of the distal end tip 32, and the flat-plate portion 1a is displaced so as to approach the sticking member 2. Caused by this displacement, the flat-plate portion 1a presses the atrial septum secundum M1 toward the foramen ovale valve M2, the positions of the atrial septum secundum M1 and the foramen ovale valve M2 in the thickness direction, in other words the positions thereof in the forward & backward direction in the operation state, are fixed, and as shown in FIG. 17, there is obtained a state in which the atrial septum secundum M1 and the foramen ovale valve M2 exist between the sandwich member 1 and the sticking member 2.

At this stage, in order to release the lock of the second lock portion R2 in the lock & unlock mechanism 102 shown in FIG. 9, 10, if the pusher piece 109 is pressed and the lock of the main steering rod 7a is released, pressurization of the first elastic wire 66 and the second elastic wire 67 by the main steering rod 7a and the bump member 68 disappears, and the first elastic wire 66 and the second elastic wire 67 get into a linearly extended state caused by their own elastic force. In this state, as shown in FIG. 12, when the slide portion 100 is steered so as to move backward, the whole positioning hold means 60 is withdrawn into the inside of the lumen L5 of the catheter 30 through the main tube 63. As shown in FIG. 12B, when the "OK" indicating portion H6 appears on the window 73, it can be understood that the withdrawal has terminated.

On the other hand, in the hand-side steering unit 70, as shown in FIG. 7, also the terminal 83 mounted on the main tube 63 moves backward and contacts the member 85, and there is obtained an electrically conductive state between the sandwich member 1 and the input connector 75. Then, this contact member 85 has a conductable range X and within this conductable range X, the cutout portion 41 of the guide bar 40A coincides with the connection hole 74 and the output connector 87 becomes connectable with the input connector 75 for the first time. More specifically, due to the conductable range X, individual differences in the thickness and the shape of the foramen ovale valve M2 or the like are absorbed and concurrently, due to the existence of the cutout portion 41, there is obtained a state in which connection between the output connector 87 and the input connector 75 becomes possible only within the conductable range X.

### (5) Connection Process

In other words, it happens that with respect to the moving-back of the slide portion 100 at this stage, the sandwiching of the biological tissue M and the contact state of the terminal 83 and the contact member 85 are carried out all at once. Furthermore, the terminal 81 on the sticking member 2 side and the contact member 84 get into an electrically conductable state in advance, so that both of the sandwich member 1 and the sticking member 2 get into a state in which they can be supplied with electric energy.

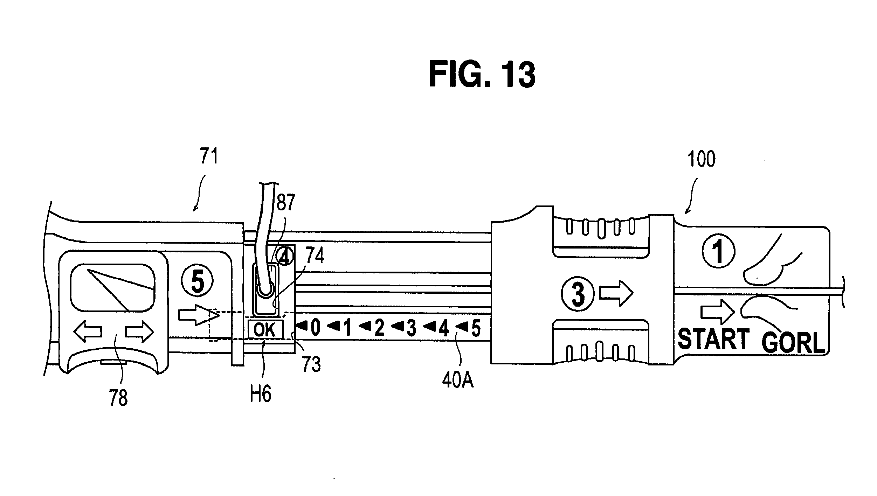

Then, as shown in FIG. 12B, there exists a state in which the "OK" indicating portion H6 appears on the window 73, so that the operator can understand that the output connector 87 is allowed to be connected to the input connector 75 (see FIG. 13) . When the output connector 87 is connected to the input connector 75, there is obtained a state in which the power supply from the energy supply means 20 is possible.

Note that in this state, if it is constituted such that the outer circumferential surface of the output connector 87 contacts the side surface of the cutout portion 41 of the guide bar 40A, the sliding of the guide bar 40A is suppressed by a frictional force. More specifically, the output connector 87 and the cutout portion 41 function as fixing means by which the sandwich member 1 (heating unit) is fixed to a constant position and the movement of the sandwich member 1 after getting into an electrically conductable state is suppressed, and it is possible to maintain a desirable sandwiching state. However, it is not necessarily required to employ a constitution in which the outer circumferential surface of the output connector 87 contacts the side surface of the cutout portion 41 of the guide bar 40A.

Thereafter, by operating the switch SW, a predetermined electric energy controlled by the control unit 22 is supplied to the sandwich member 1 and the sticking member 2 through the operation cords 7b, 7c and the atrial septum secundum M1 and the foramen ovale valve M2 are heated.

When the heating continues while maintaining the fusion temperature, the tissues of the atrial septum secundum M1 and the foramen ovale valve M2 melt and are fused mutually by adhesive factors such as collagen, elastin or the like. The control unit 22 of the electric energy controls the output power to be low, thereby making attachment of thrombi difficult, so that even if a portion of the sandwich member 1 and the sticking member 2 is exposed in the blood, attachment of the thrombus (thrombi) to the sandwich member 1 and the sticking member 2 can be prevented. The thrombi become a cause of cerebral infarction, myocardial infarction or the like, so that by suppressing the occurrence of thrombi more reliably, it is possible to improve the safety of the device.

### (6) Sticking Unit Moving-back Process

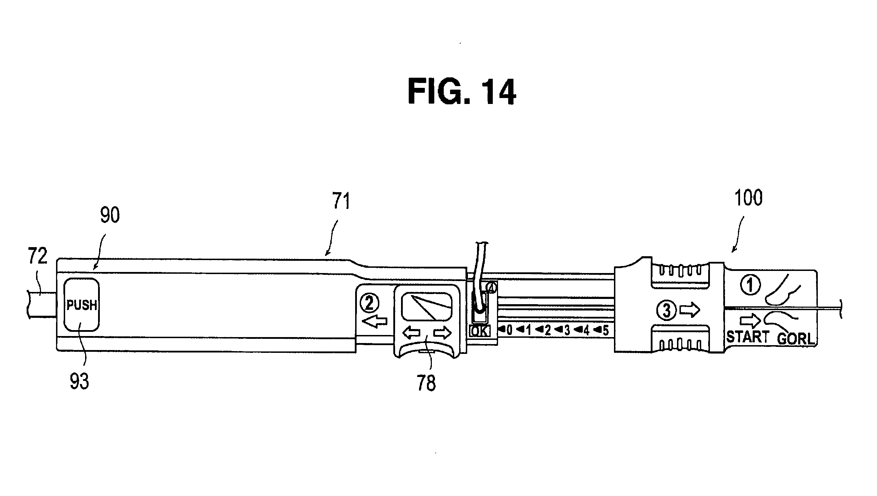

When the fusion is completed, the needle steering lever 78 shown in FIG. 13 is moved backward in accordance with the indication of an arrow indicated in the vicinity of the number (5) and is brought into the state of FIG. 14, and the sticking member 2 is housed inside the distal end tip 32. Thus, the terminal 81 which moves together with the needle steering lever 78 leaves from the contact member 84 (see FIG. 5) and the electrically conductable state with respect to the clamping means K is released. Thereafter, the output connector 87 is removed from the input connector 75. Then, the push button 93 of the interlock mechanism 90 is pressed and by releasing the interlock between the Y connector 72 and the main body portion 71, the interlock between the guiding catheter 31 and the main body portion 71 is released, and when the main body portion 71 is moved backward so as to leave from the living body, the device is pulled out with the guiding catheter 31 serving as a guide. Thereafter, when the guiding catheter 31 is pulled out from the living body, the procedure is completed.

Note that the present invention is not to be limited only by the exemplified embodiments mentioned above and it is possible to employ various modifications by persons skilled in the art within the technical ideas of the present invention.

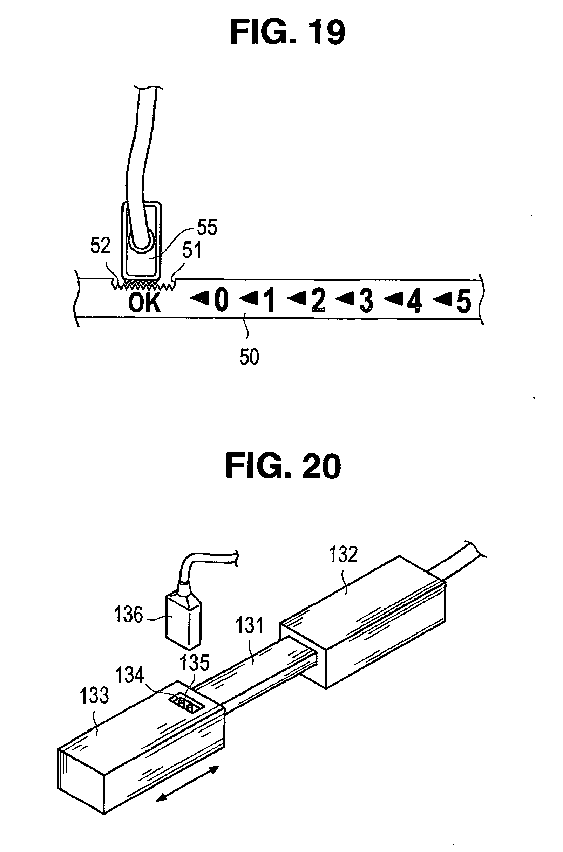

FIG. 19 shows another example of this exemplified embodiment and there is formed a notch 52 continuous to a side surface of the cut portion 51 of the guide bar 50, in which it is possible to use an output connector 55 having an exterior shape corresponding to the shape of this notch 52. If such a shape is employed, it becomes possible for the output connector 87 to be connected to the input connector 75 in an arbitrary position within the conductable range X of the contact member 85 and concurrently, after the output connector 87 is once connected to the input connector 75, the guide bar 50 becomes unslidable, so that it is possible to reliably suppress the movement of the sandwich member 1 after getting into an electrically conductable state and a desirable sandwiching state can be maintained. More specifically, the notch 52 functions as fixing means by which the slide portion 100 is fixed with respect to the main body portion 71, there is no limitation on the configuration thereof as long as the function as the fixing means is achieved by a mechanism in which the output connector 87 and the guide bar 50 contact each other.

Also, the guide bar 40A in this exemplified embodiment is fixed to the slide portion 100 on the proximal side and slides with respect to the main body portion 71, but it is also possible to employ a constitution in which it is fixed to the main body portion 71 on the distal side and slides with respect to the slide portion 100. Also, it is allowed for the input connector 75 to be provided at the slide portion 100, not at the main body portion 71.

Also, as the connection adjusting unit which makes connection between the output connector 87 and the input connector 75 possible, instead of the cutout portion 41 formed as a notch at the edge of the guide bar 40A, it is also possible to apply a through-hole which passes through the guide bar 40A.

Also, if the connection of the output connector 87 to the input connector 75 can be controlled by the guide bar 40A (guide unit) including the cutout portion 41 (or through-hole), it is allowed for the connection hole 74 corresponding to the exterior shape of the output connector 87 to be not necessarily provided.

Also, with respect to the output connector 87 and the input connector 75, if they are connectable electrically, embodiments thereof are not limited.

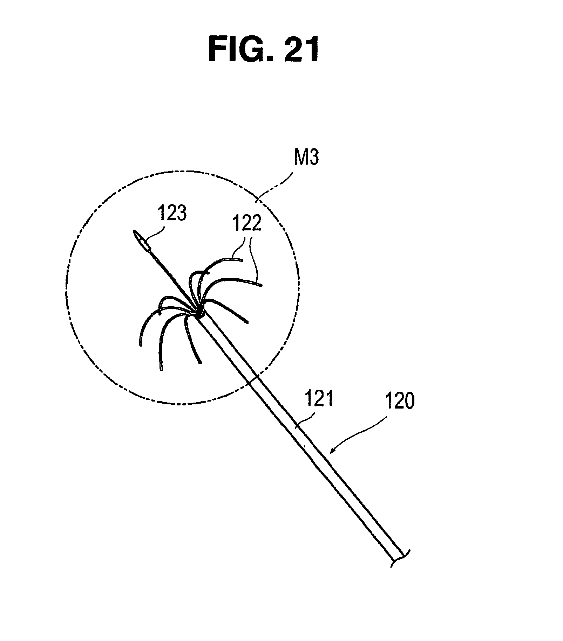

Also, FIG. 21 shows a schematic view of another exemplified embodiment to which the present invention is applied and there is employed a constitution in which a guide bar 131 is fixed to a main body portion 132 (or slide portion) and is slidable inside a slide portion 133 (or main body portion), and there is provided an input connector 134 on the guide bar 131 (guide unit) . At the slide portion 133, there is provided a connection window 135 (connection adjusting unit) in the form of a through-hole (or notch) which exposes the guide bar 131 which slides in the inside thereof. There is employed a constitution in which the connection window 135 coincides with the input connector 134 of the guide bar 131 when the heating unit is moved to the position at which the biological tissue is heatable after the slide portion 133 is advanced and retracted with respect to the main body portion 132. By employing such a constitution, when the heating unit is moved to the position at which the biological tissue is heatable, it is possible to obtain a state in which the output connector 136 is connected with the input connector 134 through the connection window 135 and electric energy can be supplied to the heating unit.

Also, in this exemplified embodiment, it was explained with respect to a device used for the treatment for closing the defect of the PFO, but the present invention is not to be limited only by this and it is usable also in case of closing a path-shaped defect such as a left-atrial-appendage (Left Atrial Appendage) closing device or in a case in which a biological tissue M at a predetermined region is necrotized thermally. In addition, as shown in FIG. 21, the present invention can be applied also to a medical device 120 or the like which is provided with a plurality of electrodes 122 protruding so as to be opened outward from the lumen inside the catheter 121 and an excision probe 123, and which applies electric energy from the electrode 122 after the electrodes 122 are protruded and opened outward inside a biological tissue such, for example, as a liver tissue M3 and the like.

### DESCRIPTION OF REFERENCE NUMERALS

1: sandwich member (heating unit),
2: sticking member (heating unit),
20: energy supply means,
30: catheter,
40A, 50, 131: guide bar (guide unit),
41, 51 cutout portion (connection adjusting unit),
52: notch (fixing means),
55, 87, 136: output connector,
70: hand-side steering unit,
71, 132: main body portion,
75, 134: input connector,
84: contact member (switch),
85: contact member (switch),
100, 133: slide portion,
121: catheter,
122: electrode (heating unit),
135: connection window (connection adjusting unit),
H6: indicating portion,
K: clamping means (heating unit),
M: biological tissue, and
X: conductable range.

## Claims

1. A medical device including a heating unit (1, 2, 122 K) provided on a distal side of a catheter (121) to heat a biological tissue and a hand-side steering unit (70) provided at a proximal portion of the catheter (121) to steer advance and retraction operation with respect to the catheter (121) at the heating unit (1, 2, 122 K),
the hand-side steering unit (70) comprises:
a main body portion (71, 132) interlocked with the catheter (121);
a slide portion (100, 33) which is interlocked with the heating unit (1, 2, 122 K) and which is approachable and separable with respect to the main body portion (71, 132);
a guide unit (40 A, 50, 131) which is interlocked movably with one of the main body portion (71, 132) and the slide portion (100, 133) and also which is fixed on the other thereof; and
an input connector (75, 134) which is provided at the main body portion (71, 132) or, the slide portion (100, 133), which is electrically connected with the heating unit (1,2, 122 K) and also with which an output connector (55, 87, 136) for supplying electric energy is connectable, **characterized in that**
the guide unit (40 A, 50, 131), is arranged at a position for hindering connection between the input connector (75, 134) and the output connector (55, 87, 136) and also, there is provided a connection adjusting unit (41, 51) which is a notch (52) or a through-hole for enabling connection between the input connector (75, 134) and the output connector (55, 87, 136) when the heating unit (1, 2, 122 K) moved to a position at which the biological tissue is heatable by moving the slide portion (100, 133) with respect to the main body portion (71, 132);
wherein the connection adjusting unit (41, 51) is formed on the guide unit (40 A, 50, 131); and wherein an outer circumferential surface of the outer connector contacts a side surface of the connection adjusting unit (41, 51) when the heating unit (1,2, 122 K) moved to the position at which the biological tissue is heatable by moving the slide portion (100, 133) with respect to the main body portion (71, 132).

2. The medical device according to claim 1, **characterized in that** the connection adjusting unit (41, 51) has a size allowing connection between the input connector (75, 134) and the output connector (55, 87, 136) when the slide portion (100, 133) lies in a relative position within a certain range with respect to the main body portion (71, 132).

3. The medical device according to claim 1 or 2, **characterized in that** at the main body portion (71, 132), at the slide portion (100, 133) or at the guide unit (40 A, 50, 131), there is provided a switch (84, 85) for carrying out electrical connection or release between the heating unit (1, 2, 122 K) and the input connector (75, 134) by the movement of the slide portion (100, 133); and corresponding to the position of the slide portion (100, 133) at which electrical connection is made by the switch (84, 85), the connection adjusting unit (41, 51) is provided such that the connection between the input connector (75, 134) and the output connector (55, 87, 136) becomes possible.

4. The medical device according to any one of claims 1 to 3, **characterized in that** for the connection adjusting unit (41, 51) and the output connector (55, 87, 136), there is provided fixing means for fixing the movement of the slide portion (100, 133) with respect to the main body portion (71, 132) by being contacted with each other when the output connector (55, 87, 136) is connected to the input connector (75, 134).

5. The medical device according to any one of claims 1 to 4, **characterized in that** for the guide unit (40 A, 50, 131), there is provided an indicating portion (H6) in which there is a notification so as to coincide with a predetermined position of the main body portion (71, 132) or of the slide portion (100, 133) in a state in which connection between the input connector (75, 134) and the output connector (55, 87, 136) is possible.

## Patentansprüche

1. Medizinische Vorrichtung, die eine Heizeinheit (1, 2, 122 K), die auf einer distalen Seite eines Katheters (121) bereitgestellt wird, um ein biologisches Gewebe zu erhitzen, und eine handseitige Steuerungseinheit (70), die an einem proximalen Abschnitt des Katheters (121) bereitgestellt wird, um eine Vorschub- und Rückzugsbedienung in Bezug auf den Katheter (121) an der Heizeinheit (1, 2, 122 K) zu steuern, umfasst,
wobei die handseitige Steuerungseinheit (70) Folgendes umfasst:
einen Hauptkörperabschnitt (71, 132), der mit dem Katheter (121) gekoppelt ist,
einen Schieberabschnitt (100, 133), der mit der Heizeinheit (1, 2, 122 K) gekoppelt ist und der in Bezug auf den Hauptkörperabschnitt (71, 132) anzunähern und zu trennen ist,
eine Führungseinheit (40 A, 50, 131), die beweglich mit dem einen von dem Hauptkörperabschnitt (71, 132) und dem Schieberabschnitt (100, 133) gekoppelt ist und
die ebenfalls an dem anderen von denselben befestigt ist, und
einen Eingangsverbinder (75, 134), der an dem Hauptkörperabschnitt (71, 132) oder dem Schieberabschnitt (100, 133) bereitgestellt wird, der elektrisch mit der Heizeinheit (1, 2, 122 K) verbunden ist und mit dem ebenfalls ein Ausgangsverbinder (55, 87, 136) zum Zuführen von Elektroenergie verbunden werden kann, **dadurch gekennzeichnet, dass** die Führungseinheit (40 A, 50, 131) bei einer Position zum Verhindern einer Verbindung zwischen dem Eingangsverbinder (75, 134) und dem Ausgangsverbinder (55, 87, 136) angeordnet ist und ebenfalls eine Verbindungseinstellungseinheit (41, 51) bereitgestellt wird, die eine Kerbe (52) oder ein Durchgangsloch ist, um eine Verbindung zwischen dem Eingangsverbinder (75, 134) und dem Ausgangsverbinder (55, 87, 136) zu ermöglichen, wenn die Heizeinheit (1, 2, 122 K) durch das Bewegen des Schieberabschnitts (100, 133) in Bezug auf den Hauptkörperabschnitt (71, 132) zu einer Position bewegt wird, bei der das biologische Gewebe erhitzt werden kann,
wobei die Verbindungseinstellungseinheit (41, 51) an der Führungseinheit (40 A, 50, 131) geformt ist und wobei eine Außenfläche des äußeren Verbinders eine Seitenfläche des Verbindungseinstellungseinheit (41, 51) berührt, wenn die Heizeinheit (1, 2, 122 K) durch das Bewegen des Schieberabschnitts (100, 133) in Bezug auf den Hauptkörperabschnitt (71, 132) zu einer Position bewegt wird, bei der das biologische Gewebe erhitzt werden kann.

2. Medizinische Vorrichtung nach Anspruch 1, **dadurch gekennzeichnet, dass** die Verbindungseinstellungseinheit (41, 51) eine Größe hat, die eine Verbindung zwischen dem Eingangsverbinder (75, 134) und dem Ausgangsverbinder (55, 87, 136) ermöglicht, wenn der Schieberabschnitt (100, 133) in einer relativen Position innerhalb eines bestimmten Bereichs in Bezug auf den Hauptkörperabschnitt (71, 132) liegt.

3. Medizinische Vorrichtung nach Anspruch 1 oder 2, **dadurch gekennzeichnet, dass** an dem Hauptkörperabschnitt (71, 132), an dem Schieberabschnitt (100, 133) oder an der Führungseinheit (40 A, 50, 131) ein Schalter (84, 85) bereitgestellt wird, um durch die Bewegung des Schieberabschnitts (100, 133) eine elektrische Verbindung oder Trennung zwischen der Heizeinheit (1, 2, 122 K) und dem Eingangsverbinder (75, 134) auszuführen, und entsprechend der Position des Schieberabschnitts (100, 133), bei der die elektrische Verbindung durch den Schalter (84, 85) hergestellt wird, die Verbindungseinstellungseinheit (41, 51) derart bereitgestellt wird, dass die Verbindung zwischen dem Eingangsverbinder (75, 134) und dem Ausgangsverbinder (55, 87, 136) möglich wird.

4. Medizinische Vorrichtung nach einem der Ansprüche 1 bis 3, **dadurch gekennzeichnet, dass** für die Verbindungseinstellungseinheit (41, 51) und den Ausgangsverbinder (55, 87, 136) Fixierungsmittel bereitgestellt werden, um die Bewegung des Schieberabschnitts (100, 133) in Bezug auf den Hauptkörperabschnitt (71, 132) dadurch zu fixieren, dass sie miteinander in Berührung gebracht werden, wenn der Ausgangsverbinder (55, 87, 136) mit dem Eingangsverbinder (75, 134) verbunden wird.

5. Medizinische Vorrichtung nach einem der Ansprüche 1 bis 4, **dadurch gekennzeichnet, dass** für die Führungseinheit (40 A, 50, 131) ein Anzeigeabschnitt (H6) bereitgestellt wird, in dem es eine Mitteilung gibt, die in einem Zustand, in dem eine Verbindung zwischen dem Eingangsverbinder (75, 134) und dem Ausgangsverbinder (55, 87, 136) möglich ist, mit einer vorbestimmten Position des Hauptkörperabschnitts (71, 132) oder des Schieberabschnitts (100, 133) übereinstimmt.

## Revendications

1. Dispositif médical comprenant une unité chauffante (1, 2, 122 K) placée sur un côté distal d'un cathéter (121) pour chauffer un tissu biologique et une unité de manipulation côté main (70) placée au niveau d'une partie proximale du cathéter (121) pour commander une opération d'avance et de rétraction par rapport au cathéter (121) au niveau de l'unité chauffante (1, 2, 122 K),
l'unité de manipulation côté main (70) comprend :
une partie de corps principal (71, 132) verrouillée réciproquement avec le cathéter (121) ;
une partie coulissante (100, 133) qui est verrouillée réciproquement avec l'unité chauffante (1, 2, 122 K) et qui peut s'approcher et se séparer de la partie de corps principal (71, 132) ;
une unité de guidage (40 A, 50, 131) qui est verrouillée réciproquement de façon mobile avec un élément parmi la partie de corps principal (71, 132) et la partie coulissante (100, 133) et qui est en outre fixée sur l'autre de ces éléments ; et
un connecteur d'entrée (75, 134) qui est placé sur la partie de corps principal (71, 132) ou la partie coulissante (100, 133), qui est connecté électriquement à l'unité chauffante (1, 2, 122 K) et auquel peut être connecté un connecteur de sortie (55, 87, 136) pour fournir de l'énergie électrique, **caractérisé en ce que** :
l'unité de guidage (40 A, 50, 131) est placée en une position destinée à gêner la connexion entre le connecteur d'entrée (75, 134) et le connecteur de sortie (55, 87, 136) et en outre, il est prévu une unité de réglage de connexion (41, 51) qui est une encoche (52) ou un trou traversant pour permettre la connexion entre le connecteur d'entrée (75, 134) et le connecteur de sortie (55, 87, 136) quand l'unité chauffante (1, 2, 122 K) est déplacée jusqu'à une position dans laquelle le tissu biologique peut être chauffé en déplaçant la partie coulissante (100, 133) par rapport à la partie de corps principal (71, 132) ;
dans lequel l'unité de réglage de connexion (41, 51) est formée sur l'unité de guidage (40 A, 50, 131) ; et
dans lequel une surface circonférentielle extérieure du connecteur extérieur est en contact avec une surface latérale de l'unité de réglage de connexion (41, 51) quand l'unité chauffante (1, 2, 122 K) est déplacée jusqu'à la position dans laquelle le tissu biologique peut être chauffé en déplaçant la partie coulissante (100, 133) par rapport à la partie de corps principal (71, 132).

2. Dispositif médical selon la revendication 1, **caractérisé en ce que** l'unité de réglage de connexion (41, 51) a une taille qui permet la connexion entre le connecteur d'entrée (75, 134) et le connecteur de sortie (55, 87, 136) quand la partie coulissante (100, 133) se trouve dans une position relative dans un certain intervalle par rapport à la partie de corps principal (71, 132).

3. Dispositif médical selon la revendication 1 ou 2, **caractérisé en ce que** sur la partie de corps principal (71, 132), sur la partie coulissante (100, 133) ou sur l'unité de guidage (40 A, 50, 131) se trouve un interrupteur (84, 85) pour réaliser une connexion électrique ou une séparation entre l'unité chauffante (1, 2, 122 K) et le connecteur d'entrée (75, 134) par le mouvement de la partie coulissante (100, 133) ; et en correspondance avec la position de la partie coulissante (100, 133) à laquelle la connexion électrique est réalisée par l'interrupteur (84, 85), l'unité de réglage de connexion (41, 51) est placée de telle manière que la connexion entre le connecteur d'entrée (75, 134) et le connecteur de sortie (55, 87, 136) devient possible.

4. Dispositif médical selon l'une quelconque des revendications 1 à 3, **caractérisé en ce que** pour l'unité de réglage de connexion (41, 51) et le connecteur de sortie (55, 87, 136), il est prévu un moyen de fixation pour fixer le mouvement de la partie coulissante (100, 133) par rapport à la partie de corps principal (71, 132) en étant mises en contact l'une avec l'autre quand le connecteur de sortie (55, 87, 136) est connecté au connecteur d'entrée (75, 134).

5. Dispositif médical selon l'une quelconque des revendications 1 à 4, **caractérisé en ce que** pour l'unité de guidage (40 A, 50, 131), il est prévu une partie de signalisation (H6) dans laquelle se trouve un repère destiné à coïncider avec une position prédéterminée de la partie de corps principal (71, 132) ou de la partie coulissante (100, 133) dans un état dans lequel la connexion entre le connecteur d'entrée (75, 134) et le connecteur de sortie (55, 87, 136) est possible.
